(19) Europäisches Patentamt
European Patent Office
Office européen des brevets

(11) EP 4 036 139 A1

# (12) EUROPEAN PATENT APPLICATION

(43) Date of publication:
**03.08.2022 Bulletin 2022/31**

(21) Application number: **21154532.2**

(22) Date of filing: **01.02.2021**

(51) International Patent Classification (IPC):
***C08G 18/76*** *(2006.01)* ***C07C 263/18*** *(2006.01)*
***C08J 3/24*** *(2006.01)* ***C08K 5/14*** *(2006.01)*
***C08L 75/00*** *(2006.01)* ***C08L 75/14*** *(2006.01)*

(52) Cooperative Patent Classification (CPC):
**C08G 18/7664; C07C 263/18; C08K 5/14; C08L 75/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Covestro Deutschland AG**
**51373 Leverkusen (DE)**

(72) Inventor: **The designation of the inventor has not yet been filed**

(74) Representative: **Levpat**
**c/o Covestro AG**
**Gebäude 4825**
**51365 Leverkusen (DE)**

## (54) A METHOD FOR STABLY STORING AN ISOCYANATE COMPOSITION

(57) The present invention relates to a method for stably storing an isocyanate composition, the stably stored isocyanate composition, and a polyurethane resin prepared from the isocyanate composition.

EP 4 036 139 A1

## Description

### Technical Field

The present invention relates to a method for stably storing an isocyanate composition, and a stably stored isocyanate composition.

### Background Technology

We have found that in the presence of a free-radical initiator, an isocyanate composition will not be stably stored because of increase of the viscosity. It is not yet known in the art how to make the viscosity of the isocyanate composition stable , so that the corresponding isocyanate compositions are stably stored.
CN101035825A discloses a method for manufacturing the polyisocyanate/polysilicic acid based resin with a wide variability of processability and curing times by reacting one or more polyisocyanates with water glass, optionally in the presence of one or more additives and/or auxiliaries for conventionally preparing the polyisocyanate/polysilicic acid based resin.
CN102781989A discloses a method of associating an iron contaminant with a beta-dicarbonyl substance in an isocyanate composition containing a polymeric diphenylmethane diisocyanate (PMDI). The association of the iron contaminant with the beta-dicarbonyl substance minimizes the catalytic effect of the iron contaminant when the isocyanate composition is reacted with a polyol to form a polyurethane.
Despite the above disclosures, there is still a great need in the art for a method of stably storing an isocyanate composition.

### Summary of the Invention

In one aspect of the present invention, there is provided a method for stably storing an isocyanate composition comprising the following components:

A1) one or more polyisocyanates;

A2) at least one free-radical initiator;

The method is to add A3) a beta-dicarbonyl compound to the isocyanate composition.
Preferably, said polyisocyanate is selected from aromatic isocyanates, preferably diphenylmethane diisocyanate, toluene diisocyanate, diphenylmethane diisocyanate or a combination thereof, particularly preferably diphenylmethane diisocyanate or (poly)diphenylmethane diisocyanate.
Preferably, the content of the A2) free-radical initiator is 0.1-5 wt%, preferably 0.2-4 wt%, more preferably 0.4-3 wt%, based on 100wt% of the total weight of the isocyanate composition.
Preferably, the content of said A3) beta-dicarbonyl compound is 0.005-2 wt%, preferably 0.009-1.8 wt%, more preferably 0.012-1.8 wt%, particularly preferably 0.012-0.048 wt%, based on 100wt% of the total weight of the isocyanate composition.
Optionally, the beta-dicarbonyl compound has a structure in which two carbonyl groups (C=O) are separated by one carbon atom, can be either the carbonyl groups in positions 1,3 according to the IUPAC nomenclature, or two carbonyl groups (C=O) in a larger molecule not located in positions 1,3 as defined according to the IUPAC nomenclature, but still separated by one carbon atom. The beta-dicarbonyl compound can be selected from nonane-4,6-dione, hexane-2,4-dione, 5-methylhexane-2,4-dione, acetylacetone, 1-phenyl-1,3-butanedione, 3,3-dimethylpentane-2,4-dione or mixtures thereof, preferably acetylacetone, hexane-2,4-dione or mixtures thereof, more preferably acetylacetone.
Preferably, the A2) free-radical initiator is selected from peroxides and/or azo compounds.
Preferably, the peroxide is selected from ketone peroxides, carbonate peroxides, acyl peroxides, ester peroxides, hydrogen peroxide, alkyl peroxides or any combination thereof.
Preferably, said azo compound is selected from azodiisobutyronitrile, azodiisoheptylonitrile or any combination thereof.
Preferably, compared to the isocyanate composition not containing the beta-dicarbonyl compound, the isocyanate composition containing the beta-dicarbonyl compound has a prolonged stable storage time at 50°C of ≥ 7 days, preferably ≥ 10 days, more preferably ≥ 14 days.
In a further aspect of the present invention, there is provided an isocyanate composition, comprising:

A1) one or more polyisocyanates;

A2) at least one free-radical initiator;

A3) at least one beta-dicarbonyl compound.

Preferably, the content of said A3) beta-dicarbonyl compound is 0.005-2 wt%, preferably 0.012-1.8 wt%, more preferably 0.012-0.048 wt%, based on 100wt% of the total weight of the isocyanate composition.

Preferably, compared to the isocyanate composition not containing the beta-dicarbonyl compound, the isocyanate composition containing the beta-dicarbonyl compound has a prolonged stable storage time at 50°C of ≥ 7 days, preferably ≥ 10 days, more preferably ≥ 14 days.

In a further aspect of the present invention, there is provided a method for preparing a polyurethane resin, reacting a polyurethane reaction system comprising the following components to obtain the polyurethane resin:

Component A), comprising: the aforementioned isocyanate composition of the present invention;

Component B), comprising:

B1) one or more organic polyols, the content of the organic polyol is 21-60 wt%, based on 100 wt% of the total weight of the polyurethane reaction system;

B2) one or more compounds having the structure of formula (I)

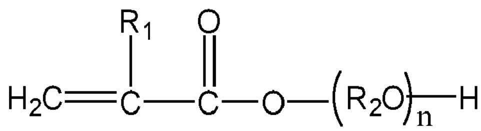

I

,

Wherein, $R_1$ is selected from hydrogen, methyl or ethyl; $R_2$ is selected from an alkylene group having 2-6 carbon atoms, 2,2-bis(4-phenylene)-propane, 1,4-bis(methylene)benzene, 1,3-bis(methylene)benzene, 1,2-bis(methylene)benzene; n is an integral number selected from 1-6.

Preferably, said B1) organic polyol is selected from those having the functionality of 1.7-6, preferably 1.9-4, more preferably 1.9-2.8 and the hydroxyl value of 150-1100 mgKOH/g.

Preferably, the content of said B2) is 4-33 wt%, based on 100 wt% of the total weight of the polyurethane reaction system.

Preferably, the component B2) is selected from: hydroxyethyl methacrylate, hydroxypropyl methacrylate, hydroxybutyl methacrylate, hydroxyethyl acrylate, hydroxypropyl acrylate, hydroxybutyl acrylate or a combination thereof.

Preferably, the polyurethane reaction system has a pot-life at 35°C of ≥ 60 minutes, preferably ≥ 65 minutes, more preferably ≥ 70 minutes.

In a further aspect of the present invention, there is provided a polyurethane resin, which is obtained by the method for preparing a polyurethane resin according to the present invention.

In a further aspect of the present invention, there is provided a polyurethane product comprising the polyurethane resin according to claim 16, characterized in that the polyurethane product is selected from cable trays, frames of doors, windows and curtain walls, frames of ladders, tent poles or pipes, anti-glare shields, floors, sucker rods, telegraph poles and cross arms, guardrails, gratings, architectural sectional materials, container sectional materials and sheets, bike racks, fishing rods, cable cores, insulator core rods, antenna housings, single-layer or sandwiched continuous sheets and blade shells, webs, spar caps, main spars, auxiliary spars and blade roots of turbine fan blades.

Directly mixing the free-radical initiator into the isocyanate has an adverse effect on the transport and long-term storage of the isocyanate mixture. Especially under the condition of direct sunlight at high temperature in summer, the internal temperature of a common van-type transport vehicle in a sealed environment can reach 50-60°C, so that the viscosity of the isocyanate will be increased more quickly, and the quality of the isocyanate product is influenced. During some long maritime transports, ships may travel near the tropical equator for several weeks, and under such long term high temperature conditions, it is more difficult to ensure the quality of the isocyanate mixture to which the free radical initiator is added. Surprisingly, we have found that the addition of an appropriate amount of a beta-dicarbonyl compound to the isocyanate mixture is effective in prolonging the storage time without having to worry about the quality problem during the transportation process even under tropical weather conditions. The method of the present invention simply, economically and efficiently realizes the stable storage of the isocyanate composition, successfully prolongs the

shelf life of the isocyanate product, thereby being able to simplify the production process of the polyurethane resin, in particular the production process of the polyurethane resin for producing the large manufactured product such as fan blades and the like, and improve the relevant finished product rate and the production efficiency.

Detailed description of the invention

The following terms used in the present invention have the following explanations or interpretations.

Pbw, refers to the mass fraction of each component in the reaction system;

wt.%, refers to mass percent;

Functionality, refers to the value determined according to the industry formula: functionality=hydroxyl value * molecular weight/56 100; wherein the molecular weight is determined by the GPC high performance liquid chromatography;

Isocyanate index, refers to the value calculated by the following formula:

$$\text{Isocyanate index (\%)} = \text{the mole number of isocyanate groups (NCO groups) in Component A/the mole number of isocyanate group-reactive groups in Component B} \times 100\%.$$

The method for stably storing the isocyanate composition provided by the present invention comprises the following components:

A1) one or more polyisocyanates;

A2) at least one free-radical initiator;

The method is to add A3) a beta-dicarbonyl compound to the isocyanate composition.

Furthermore, as described previously, the present invention also provides the aforementioned isocyanate composition, the polyurethane reaction system, the method for preparing a polyurethane resin, and the like.

Any organic polyisocyanate, comprising aromatic, aliphatic and alicyclic polyisocyanates and a combination thereof, can be used in the above-mentioned method of the present invention. The polyisocyanate may be represented by a general formula R(NCO)n, wherein R represents an aliphatic hydrocarbon group having 2-18 carbon atoms, an aromatic hydrocarbon group having 6-15 carbon atoms, and an araliphatic hydrocarbon group having 8-15 carbon atoms, and n = 2-4.

Usable polyisocyanates comprise, preferably, but are not limited to ethylene diisocyanate, tetramethylene 1,4-diisocyanate, hexamethylene diisocyanate (HDI), dodecylene 1,2-diisocyanate, cyclobutane-1,3-diisocyanate, cyclohexane-1,3-diisocyanate, cyclohexane-1,4-diisocyanate, 1-isocyanato-3,3,5-trimethyl-5-isocyanatomethylcyclohexane, hexahydrotoluene-2,4-diisocyanate, hexahydrophenyl-1,3-diisocyanate, hexahydrophenyl-1,4-diisocyanate, perhydro-diphenylmethane-2,4-diisocyanate, perhydro-diphenylmethane-4,4-diisocyanate, phenylene-1,3-diisocyanate, phenylene-1,4-diisocyanate, stilbene-1,4-diisocyanate, 3,3-dimethyl-4,4-diphenyldiisocyanate, toluene-2,4-diisocyanate (TDI), toluene-2,6-diisocyanate (TDI), diphenylmethane-2,4'-diisocyanate (MDI), diphenylmethane-2,2'-diisocyanate (MDI), diphenylmethane-4,4'-diisocyanate (MDI), mixtures of diphenylmethane diisocyanate and/or diphenylmethane diisocyanate homologues having more rings, polyphenylmethane polyisocyanate (polymeric MDI), naphthylene-1,5-diisocyanate (NDI), their isomers, and any mixture of them and their isomers.

Usable polyisocyanates also comprise isocyanates obtained by modification with a carbodiimide, an allophanate or the like, and are preferably, but not limited to diphenylmethane diisocyanate, carbodiimide modified diphenylmethane diisocyanate, their isomers, and any mixture of them and their isomers.

When used in the present invention, polyisocyanates comprise isocyanate dimers, trimers, tetramers, or a combination thereof.

In some embodiments of the present invention, the isocyanate is selected from aromatic isocyanates, preferably diphenylmethane diisocyanate, toluene diisocyanate, diphenylmethane diisocyanate or a combination thereof, particularly preferably diphenylmethane diisocyanate or (poly)diphenylmethane diisocyanate.

In a preferred embodiment of the present invention, the polyisocyanate component is selected from polymeric MDIs.

The NCO content of the organic polyisocyanate of the present invention is 20-33 wt%, preferably 25-32 wt%. The NCO content is determined by GB/T12009.4-2016.

The organic polyisocyanates can also be used in the form of polyisocyanate prepolymers. These polyisocyanate prepolymers can be obtained by reacting an excess of the above-mentioned organic polyisocyanate with a compound having at least two isocyanate-reactive groups at a temperature of for example 30-100°C, preferably about 80°C. The NCO content of the polyisocyanate prepolymer of the present invention is 20-33 wt%. The NCO content is determined by GB/T12009.4-2016.

The free-radical initiator refers to an agent capable of generating free radicals in a free radical reaction. It is also referred to as a free radical initiator. The process of generating free radicals becomes chain initiation. The free radical initiator that can be used in the present invention includes, but is not limited to, peroxide initiators, azo initiators, redox initiators and the like, and the peroxide initiator is further divided into organic peroxide initiators and inorganic peroxide initiators.

The organic peroxide compound has a structural general formula of R-O-O-H or R-O-OR, wherein R is an alkyl group, an acyl group, a carbonate ester group and the like. It further comprises: acyl peroxides, for example: benzoyl peroxide, lauroyl peroxide; hydroperoxides, for example: cumene hydroperoxide, tert-butyl hydroperoxide; dialkyl peroxides, for example: di-tert-butyl peroxide, diisopropylbenzene hydroperoxide; ester peroxides, t-butyl peroxybenzoate, tert-butyl peroxypivalate; ketone peroxides, for example: methyl ethyl ketone peroxide, cyclohexanone peroxide; dicarbonate peroxides, for example: diisopropyl peroxydicarbonate, dicyclohexyl peroxydicarbonate. The azo initiator can be selected from azodiisobutyronitrile, azodiisoheptylonitrile or a combination thereof.

The beta-dicarbonyl compound of the present invention refers to those compounds where the two carbonyl groups in the molecule are separated by one saturated carbon atom. These two carbonyl groups may be carbonyl groups of ketones or aldehydes, or carbonyl groups of carboxylic acids or esters. They can be either the carbonyl groups in positions 1,3 according to the IUPAC nomenclature, or two carbonyl groups (C=O) in a larger molecule not located in positions 1,3 as defined according to the IUPAC nomenclature, but still separated by one carbon atom. In particular, the beta-dicarbonyl compounds may include beta-diketones, such as $CH_3COCH_2COCH_3$; beta-keto acids and esters, such as $CH_3COCH_2COOCH_3$; acids and esters thereof, for example $C_2H_5OOCCH_2COOC_2H_5$. They can also be selected from nonane-4,6-dione, hexane-2,4-dione, 5-methylhexane-2,4-dione, acetylacetone, 1-phenyl-1,3-butanedione, 3,3-dimethylpentane-2,4-dione or mixtures thereof, preferably acetylacetone, hexane-2,4-dione or mixtures thereof, more preferably acetylacetone.

Beta-dicarbonyl compounds or beta-keto ester compounds can be prepared by the Claisen condensation reaction of esters. An ester (such as ethyl acetate) is condensed under the catalysis of a strong base (such as sodium ethoxide) and acidified to obtain a beta-keto ester (ethyl acetoacetate). The malonate compound can be obtained by the reaction of alpha-halogenated acid salt with NaCN and then hydrolysis and acidification.

Acetylacetone of the present invention is also called 2,4-pentanedione. Acetylacetone can be prepared by acylating acetone with acetic anhydride in the presence of boron trifluoride, or by condensing acetone with ethyl acetate.

The polyol according to the present invention may be a polyether polyol, a polyester polyol, a polycarbonate polyol and/or a mixture thereof.

Examples of polyether polyols that can be used in the present invention are aromatic amine-initiated polyether polyols, preferably propylene oxide-based polyether polyols initiated with diphenylmethane diamine.

A part of polyether polyols useful in the present invention are selected from glycerol, propylene glycol, sucrose, sorbitol-initiated polyether polyols, more preferably a part of polyether polyols are selected from sucrose, and sorbitol-initiated, propylene oxide-based polyether polyols.

Said polyester polyol is prepared by reacting dicarboxylic acid or dicarboxylic anhydride with polyol. The dicarboxylic acid is preferably, but not limited to, an aliphatic carboxylic acid containing 2-12 carbon atoms, for example, succinic acid, malonic acid, glutaric acid, adipic acid, suberic acid, azelaic acid, sebacic acid, lauryl acid, maleic acid, fumaric acid, phthalic acid, isophthalic acid, terephthalic acid, or a mixture thereof. The dicarboxylic anhydride is preferably, but not limited to, phthalic anhydride, tetrachlorophthalic anhydride, maleic anhydride, or a mixture thereof. The polyol is preferably, but not limited to, ethylene glycol, diethylene glycol, 1,2-propylene glycol, 1,3-propylene glycol, dipropylene glycol, 1,3-methylpropylene glycol, 1,4-butylene glycol, 1,5-pentylene glycol, 1,6-hexylene glycol, neopentyl glycol, 1,10-decanediol, glycerol, trimethylolpropane or mixtures thereof. The polyester polyol also comprises a polyester polyol prepared from lactone. The polyester polyol prepared from lactone is preferably, but not limited to, a polyester polyol prepared from ε-caprolactone.

The polycarbonate polyol is preferably, but not limited to polycarbonate diol. The polycarbonate diol may be prepared by reacting a diol with a dihydrocarbyl or diaryl carbonate or phosgene. The diol is preferably, but not limited to 1,2-propylene glycol, 1,3-propylene glycol, 1,4-butylene glycol, 1,5-pentylene glycol, 1,6-hexylene glycol, diethylene glycol, trioxymethylene glycol or a mixture thereof. The dihydrocarbyl or diaryl carbonate is preferably, but not limited to, diphenyl carbonate.

Optionally, the polyurethane reaction system of the present invention also comprises B2) one or more compounds having the structure of formula (I)

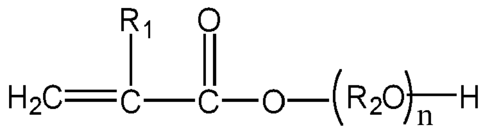

I ,

Wherein, $R_1$ is selected from hydrogen, methyl or ethyl; $R_2$ is selected from an alkylene group having 2-6 carbon atoms; n is an integral number selected from 1-6.

In a preferred embodiment of the present invention, $R_2$ is selected from ethylene, propylene, butylene, pentylene, 1-methyl-1,2-ethylene, 2-methyl-1,2-ethylene, 1-ethyl-1,2-ethylene, 2-ethyl-1,2-ethylene, 1-methyl-1,3-propylene, 2-methyl-1,3-propylene, 3-methyl-1,3-propylene, 1-ethyl-1,3-propylene, 2-ethyl-1,3-propylene, 3-ethyl-1,3-propylene, 1-methyl-1,4-butylene, 2-methyl-1,4-butylene, 3-methyl-1,4-butylene and 4-methyl-1,4-butylene, 2,2-bis(4-phenylene)-propane, 1,4-dimethylenebenzene, 1,3 -dimethylenebenzene, 1,2-dimethylenebenzene.

Preferably, said B1) is selected from an organic polyol, wherein the organic polyol is selected from those having the functionality of 1.7-6, preferably 1.9-4.5, and the hydroxyl value of 150-1100 mgKOH/g, preferably 150-550 mgKOH/g.

In a preferred embodiment of the present invention, the component B2) is selected from: hydroxyethyl methacrylate, hydroxypropyl methacrylate, hydroxybutyl methacrylate, hydroxyethyl acrylate, hydroxypropyl acrylate, hydroxybutyl acrylate or a combination thereof.

The compound of formula (I) may be prepared by conventional methods in the art, for example may be prepared by the esterification reaction of (meth)acrylic anhydride or (meth)acrylic acid, (meth)acryloyl halide compounds and $HO\text{-}(R_2O)_nH$. Those skilled in the art are familiar with these preparation methods, for example, as explained in "Manual of Polyurethane Raw Materials and Additives" (Liu Yijun, published on 1st April 2005), Chapter III, and "Polyurethane Elastomer" (Liu Houjun, published in August 2012), Chapter II. The entire contents of these documents are incorporated herein by reference.

In the method for preparing a polyurethane resin of the present invention, in the addition polymerization reaction of an isocyanate group with a hydroxyl group, the isocyanate group can be an isocyanate group contained in the organic polyisocyanate (component A), or an isocyanate group contained in the intermediate product of the reaction of the organic polyisocyanate (component A) and the organic polyol (B1) component) or B2) component, the hydroxy group can be a hydroxy group contained in the organic polyol (B1) component) or B2) component, or a hydroxy group contained in the intermediate product of the reaction of the organic polyisocyanate (component A) and the organic polyol (B1) component) or B2) component.

The free radical polymerization reaction of the present invention is an addition polymerization reaction of an olefinic bond, wherein the olefinic bond may be an olefinic bond contained in B2) component or an olefinic bond contained in the intermediate product of the reaction of B2) component and the organic polyisocyanate.

In the method for preparing a polyurethane resin of the present invention, the polyurethane addition polymerization reaction (i.e., an addition polymerization reaction of the isocyanate group and the hydroxyl group) is simultaneously performed with the free radical polymerization reaction. As known to those skilled in the art, suitable reaction conditions can be selected to allow the polyurethane addition polymerization reaction and the free radical polymerization reaction to proceed successively, but the polyurethane matrix prepared in this way is structurally different from the polyurethane resin matrix prepared by allowing the polyurethane addition polymerization reaction and the free radical polymerization reaction to proceed simultaneously, so that the prepared polyurethane composites are different in mechanical properties and manufacturability.

Optionally, the above-mentioned polyurethane reaction system can also contain auxiliaries or additives comprising, but not limited to: filler, internal release agent, flame retardant, smoke suppressant, dye, pigment, antistatic agent, antioxidant, UV stabilizer, diluent, defoamer, coupling agent, surface wetting agent, leveling agent, water-removing agent, catalyst, molecular sieve, thixotropic agent, plasticizer, foaming agent, foam stabilizing agent, foam stabilizer, free radical reaction suppressant or a combination thereof, and these component may be optionally contained in the component A) isocyanate and/or the isocyanate-reactive component namely component B) of the present invention. These components can also be stored separately as an additional component, and when used for the preparation of the polyurethane composite, are firstly mixed with the isocyanate component A) and/or the component B) of the present invention before the preparation.

In some embodiments of the present invention, the filler is selected from: aluminum hydroxide, bentonite, pulverized fuel ash, wollastonite, perlite powder, hollow microsphere, calcium carbonate, talcum powder, mica powder, porcelain clay, fumed silica, expandable microsphere, diatomite, volcanic ash, barium sulfate, calcium sulfate, glass

microsphere, stone powder, wood flour, wood chip, bamboo powder, bamboo chip, rice grain, straw chips, sorghum straw chips, graphite powder, metal powder, thermosetting composite recycled powder, plastic particle or powder, or a combination thereof. Among others, the glass microspheres can be solid or hollow.

The internal release agent that can be used in the present invention comprises any release agent conventionally used for producing polyurethanes, and examples thereof include long-chain carboxylic acids, particularly fatty acids such as stearic acid, amines of long-chain carboxylic acids such as stearic amide, fatty acid esters, metal salts of long-chain carboxylic acids such as zinc stearate, or polysiloxanes.

Examples of flame retardants that can be used in the present invention include triaryl phosphates, trialkyl phosphates, triaryl phosphates or trialkyl phosphates with halogen, melamine, melamine resins, halogenated paraffins, red phosphorus, or combinations thereof.

Other auxiliaries that can be used in the present invention include water-removing agents such as molecular sieves; defoamers such as polydimethyl siloxane; coupling agents such as mono(ethylene oxide) or organic amine functionalized trialkoxysilane or combinations thereof. Coupling agents are particularly preferably for improving the adhesion of the resin matrix to the fiber-reinforced material. Fine particle fillers, such as clay and fumed silica, are commonly used as thixotropic agent.

We have surprisingly found through repeated experiments that the method of the present invention can simply, economically and efficiently provide an isocyanate composition that can be stably stored, and effectively prolong the shelf life of the isocyanate composition. Therefore, not only the polyurethane resin with excellent quality can be prepared, but also the process can be simplified and the finished product rate and the production efficiency can be increased.

## Examples

Source of the raw materials:

Isocyanate 44V20: NCO%=30.5-32.5%, viscosity range: 160-240mPa•s@25°C, purchased from Covestro (Shanghai) Investment Co., Ltd.;

Tert-butyl peroxybenzoate: purchased from Nouryon;

Beta-dicarbonyl compound: purchased from Shanghai Lingfeng Chemical Reagent Co., Ltd;

GTS-THP gelation time determinator: purchased from Shanghai sino-lab instrument Co., Ltd.

Each of test methods in Examples are explained as follows:
NCO content, refers to the content of the NCO groups in the system, and is measured by GB/T12009.4-2016 at room temperature.

Pot-life, refers to the time from stirring the components of the reaction system uniformly until the viscosity reaches 600 mPa•S. The pot-life of the present invention is measured in the following manner: components of the polyurethane reaction system are thermostatically treated at 35°C respectively, and then mixed in the required proportion; the mixture is stirred for 1 minute until the mixture is mixed uniformly; under the temperature being maintained at 35°C, the viscosity of the mixture is measured once every 3 minutes with a viscometer, and the time required until the viscosity of the reaction system reaches 600 mPa•S, namely the pot-life, is recorded. The viscometer is a product from Brook-Field Corporation (Model Pro+DV-II).

Viscosity test: the test is performed at 25°C according to GB/T 12008.8-1992 Standard.

Stable storage time: the isocyanate composition is tested for the NCO content and the viscosity according to the above methods (the sample to be tested is tested at the corresponding temperature or after being cooled to the corresponding temperature), if the NCO content and the viscosity have no or little change, the isocyanate composition is considered to be stably stored, and the time length is recorded, i.e., the stable storage time. No or little change/reduction in the NCO content according to the present invention can mean that the NCO content is changed/reduced by not more than 5%; and it can be calculated by the following formula: the change/reduction in the NCO content by percent = (the initial NCO content - the NCO content after being stored for a period of time)/the initial NCO content. No or little change/increase in the viscosity according to the present invention can mean that the viscosity is changed/increased by not more than 50%; and it can be calculated by the following formula: the change/increase in the viscosity by percent = (the viscosity after being stored for a period of time - the initial viscosity)/the initial viscosity.

Comparative Example 1

20 g (2 wt%) of t-butyl peroxybenzoate was added to 980 g of the isocyanate raw material 44V20, and the mixture was stirred uniformly to prepare an isocyanate composition containing a free-radical initiator. The composition was placed in a 50°C oven, and at set intervals (i.e., storage time), samples were taken out, and the changes of the NCO content and the viscosity were monitored overtime. The test results were shown in Table 1.

Table 1: Test results of Comparative Example 1

| Storage time at 50°C (days) | 0 | 7 | 21 | 28 | 35 | 42 |
|---|---|---|---|---|---|---|
| Viscosity at 25°C (mPa•s) | 194 | 225 | 250 | 880 | 2200 | 8900 |
| NCO content (%) | 30.21 | 30.13 | 30.10 | 29.01 | 28.19 | 27.21 |

As could be seen from the data in table 1, the viscosity of the isocyanate compositions increased significantly over time, and had already been severely unsatisfactory with respect to quality standards after four week storage at 50°C, and the NCO content also decreased accordingly, resulting in being incapable of normal use.

Example 1

20 g (2 wt%) of t-butyl peroxybenzoate was added to 980 g of the isocyanate raw material 44V20, and the mixture was stirred uniformly to prepare an isocyanate composition containing a free-radical initiator. 0.2 g (0.02 wt%) of beta-dicarbonyl compound was added to this isocyanate composition, the mixture was stirred uniformly and placed in a 50°C oven, and at set intervals (i.e., storage time), samples were taken out, and the changes of the NCO content and the viscosity were monitored over time. The test results were shown in Table 2.

Table 2: test results of Example 1

| Storage time at 50°C (days) | 0 | 7 | 21 | 28 | 35 | 42 |
|---|---|---|---|---|---|---|
| Viscosity at 25°C (mPa•s) | 185 | 198 | 220 | 231 | 234 | 245 |
| NCO content (%) | 30.11 | 30.03 | 30.00 | 29.90 | 29.93 | 29.75 |

As could be seen from the above Comparative Example 1 and Example 1, the storage and transportation stability of the isocyanate composition could be greatly improved and the shelf life of the isocyanate product could be effectively prolonged by adding a proper amount of beta-dicarbonyl compound into the isocyanate composition.

Although the preferred embodiment of the present invention has been disclosed as above, however it is not intended to limit the present invention. Anyone skilled in the art can make various changes and modifications without departing from the spirit and scope of the present invention. Therefore, the protection scope of the present invention shall be subject to the scope of the claims in the patent application.

**Claims**

1. A method for stably storing an isocyanate composition comprising the following components:

   A1) one or more polyisocyanates;
   A2) at least one free-radical initiator;

   the method is to add A3) a beta-dicarbonyl compound to the isocyanate composition.

2. The method according to claim 1, **characterized in that** said polyisocyanate is selected from aromatic isocyanates, preferably diphenylmethane diisocyanate, toluene diisocyanate, diphenylmethane diisocyanate or a combination thereof, particularly preferably diphenylmethane diisocyanate or (poly)diphenylmethane diisocyanate.

3. The method according to claim 1 or 2, **characterized in that** the content of said A2) free-radical initiator is 0.1-5 wt%, preferably 0.2-4 wt%, more preferably 0.4-3 wt%, based on 100wt% of the total weight of the isocyanate composition.

**4.** The method according to claim 1 or 2, **characterized in that** the content of said A3) beta-dicarbonyl compound is 0.005-2 wt%, preferably 0.009-1.8 wt%, more preferably 0.012-1.8 wt%, particularly preferably 0.012-0.048 wt%, based on 100wt% of the total weight of the isocyanate composition.

**5.** The method according to claim 1 or 2, **characterized in that** said A2) free-radical initiator is selected from peroxides and/or azo compounds.

**6.** The method according to claim 1 or 2, **characterized in that** compared to the isocyanate composition not containing the beta-dicarbonyl compound, the isocyanate composition containing the beta-dicarbonyl compound has a prolonged stable storage time at 50°C of ≥ 7 days, preferably ≥ 10 days, more preferably ≥ 14 days.

**7.** An isocyanate composition, comprising:

A1) one or more polyisocyanates;
A2) at least one free-radical initiator;
A3) at least one beta-dicarbonyl compound.

**8.** The isocyanate composition according to claim 7, **characterized in that** the A2) free-radical initiator is selected from peroxides and/or azo compounds.

**9.** The isocyanate composition according to claim 7 or 8, **characterized in that** the beta-dicarbonyl compound can be selected from nonane-4,6-dione, hexane-2,4-dione, 5-methylhexane-2,4-dione, acetylacetone, 1-phenyl-1,3-butanedione, 3,3-dimethylpentane-2,4-dione or mixtures thereof, preferably acetylacetone, hexane-2,4-dione or mixtures thereof, more preferably acetylacetone.

**10.** The isocyanate composition according to claim 7 or 8, **characterized in that** the content of said A3) beta-dicarbonyl compound is 0.005-2 wt%, preferably 0.012-1.8 wt%, more preferably 0.012-0.048 wt%, based on 100wt% of the total weight of the isocyanate composition.

**11.** The isocyanate composition according to claim 7 or 8, **characterized in that** compared to the isocyanate composition not containing the beta-dicarbonyl compound, the isocyanate composition containing the beta-dicarbonyl compound has a prolonged stable storage time at 50°C of ≥ 7 days, preferably ≥ 10 days, more preferably ≥ 14 days.

**12.** A method for preparing a polyurethane resin, reacting a polyurethane reaction system comprising the following components to obtain the polyurethane resin:

Component A), comprising:
The isocyanate composition according to any of claims 7-9;
Component B), comprising:

B1) one or more organic polyols, the content of the organic polyol is 21-60 wt%, based on 100 wt% of the total weight of the polyurethane reaction system;
B2) one or more compounds having the structure of formula (I)

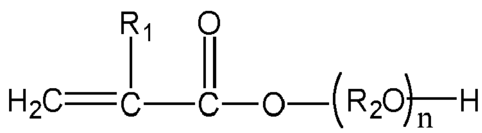

I ,

wherein, $R_1$ is selected from hydrogen, methyl or ethyl; $R_2$ is selected from an alkylene group having 2-6 carbon atoms, 2,2-bis(4-phenylene)-propane, 1,4-bis(methylene)benzene, 1,3-bis(methylene)benzene, 1,2-bis(methylene)benzene; n is an integral number selected from 1-6.

**13.** The method according to claim 12, **characterized in that** the component B2) is selected from: hydroxyethyl methacrylate, hydroxypropyl methacrylate, hydroxybutyl methacrylate, hydroxyethyl acrylate, hydroxypropyl acrylate,

hydroxybutyl acrylate or a combination thereof.

**14.** A polyurethane resin obtained by the method for preparing a polyurethane resin according to claim 12 or 13.

**15.** A polyurethane product comprising the polyurethane resin according to claim 14, **characterized in that** the polyurethane product is selected from cable trays, frames of doors, windows and curtain walls, frames of ladders, tent poles or pipes, anti-glare shields, floors, sucker rods, telegraph poles and cross arms, guardrails, gratings, architectural sectional materials, container sectional materials and sheets, bike racks, fishing rods, cable cores, insulator core rods, antenna housings, single-layer or sandwiched continuous sheets and blade shells, webs, spar caps, main spars, auxiliary spars and blade roots of turbine fan blades.

Europäisches Patentamt
European Patent Office
Office européen des brevets

# EUROPEAN SEARCH REPORT

Application Number
EP 21 15 4532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 96/30438 A1 (ARISTECH CHEMICAL CORP [US]) 3 October 1996 (1996-10-03)<br>* claim 1; tables I-V *<br>----- | 1-15 | INV.<br>C08G18/76<br>C07C263/18<br>C08J3/24<br>C08K5/14<br>C08L75/00<br>C08L75/14 |
| A | US 2007/249862 A1 (POLINVENT FEJLESZTO KIVITELEZO [HU]; NAGY GABOR [HU] ET AL.) 25 October 2007 (2007-10-25)<br>* paragraphs [0038], [0073], [0094]; claim 1; examples 24,25; table 4 * | 1-15 | |
| A,D | & CN 101 035 825 A (CONSTR RES & TECH GMBH [DE]) 12 September 2007 (2007-09-12)<br>----- | 1-15 | |

TECHNICAL FIELDS SEARCHED (IPC)

C08G
C07C
C08J
C08K
C08L

The present search report has been drawn up for all claims

1

| Place of search | Date of completion of the search | Examiner |
|---|---|---|
| Munich | 5 July 2021 | Lanz, Sandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 4532

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2021

| Patent document cited in search report | Publication date | Patent family member(s) | Publication date |
| --- | --- | --- | --- |
| WO 9630438 A1 | 03-10-1996 | AT 223939 T | 15-09-2002 |
| | | CA 2190878 A1 | 03-10-1996 |
| | | DE 69528177 T2 | 02-01-2003 |
| | | EP 0776345 A1 | 04-06-1997 |
| | | ES 2182917 T3 | 16-03-2003 |
| | | JP H10501024 A | 27-01-1998 |
| | | US 5447921 A | 05-09-1995 |
| | | US 5482648 A | 09-01-1996 |
| | | WO 9630438 A1 | 03-10-1996 |
| US 2007249862 A1 | 25-10-2007 | CN 101035825 A | 12-09-2007 |
| | | DE 112005002037 T5 | 25-10-2007 |
| | | EP 1809680 A1 | 25-07-2007 |
| | | HU 227575 B1 | 29-08-2011 |
| | | JP 2008510055 A | 03-04-2008 |
| | | US 2007249862 A1 | 25-10-2007 |
| | | WO 2006018667 A1 | 23-02-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101035825 A
- CN 102781989 A


### Non-patent literature cited in the description


- **LIU YIJUN.** Manual of Polyurethane Raw Materials and Additives. 01 April 2005
- **LIU HOUJUN.** Polyurethane Elastomer. August 2012